# EUROPEAN PATENT SPECIFICATION

(11) **EP 1 259 231 B1**
(45) Date of publication and mention of the grant of the patent: **25.05.2005**
(21) Application number: 00985618.8
(22) Date of filing: 18.12.2000
(51) Int. Cl.: A61K 31/122

(54) **NAPHTHOQUINONE DERIVATIVES AS CD45 INHIBITORS**
NAPHTHOQUINONDERIVATIVE ALS CD45 INHIBITOREN
INHIBITEURS CD45

(30) Priority: 21.12.1999 US 172786 P
(43) Date of publication of application: 27.11.2002
(73) Proprietor: AstraZeneca AB, 151 85 Södertälje (SE)
(72) Inventor: CHAPDELAINE, Marc, Jerome, Wilmington, DE 19850-5437 (US); KNAPPENBERGER, Katherine, Wilmington, DE 19850-5437 (US); STEELMAN, Gary, Wilmington, DE 19850-5437 (US); SUCHARD, Suzanne, Wilmington, DE 19850-5437 (US); SYGOWSKI, Linda, Wilmington, DE 19850-5437 (US); URBANEK, Rebecca, Wilmington, DE 19850-5437 (US); VEALE, Chris, Allan, Wilmington, DE 19850-5437 (US)
(74) Representative: Bryant, Tracey
(86) International application number: PCT/GB2000/004872
(87) International publication number: WO 2001/045681

(56) References cited:
- US-A- 5 866 569
- P. PERUMAL ET. AL.: "Oxidation with Halophenols and highly Substituted Phenols with Lead (IV) Acetate" SYNTHESIS, vol. 1980, no. 11, November 1980 (1980-11), pages 943-5, XP001026188
- A. TAKUWA ET. AL.: "The Addition of Alcohol to 1,2-Naphthoquinone Promoted by Metal Ions. A Facile Synthesis of 4-Alkoxy-1,2-Naphthoquinones" BULLETIN OF THE CHEMICAL SOCIETY OF JAPAN, vol. 59, September 1986 (1986-09), pages 2959-61, XP002142395
- T. ITAHARA: "Oxidative Coupling of Quinones and Aromatic Compounds by Palladium (II) Acetate." JOURNAL OF ORGANIC CHEMISTRY, vol. 50, 1985, pages 5546-50, XP002044654
- J.C. HENRION ET. AL.: "Condensation Indole-1,2-naphthoquinone. Recherche des Conditions Optimales dans la Préparation d'une Nouvellle Série de 4-(3-Indolyl)-1,2-naphthoquinones." BULLETIN DES SOCI T S CHIMIQUES BELGES, vol. 105, no. 7, 1996, pages 415-8, XP001026771
- A.N. GRINEV ET. AL.: "Synthesis of bis(diethylaminoethyl)-1,2-dimethyl-8-oxoi ndeno[2,1-b]pyrroloe-3,5-dicarboxylate. " CHEMISTRY OF HETEROCYCLIC COMPOUNDS (ENGLISH TRANSLATION), vol. 19, 1983, pages 876-9, XP001026781
- R.A. URBANEK ET. AL.: "Potent Reversible Inhibitors of the Protein Tyrosine Phosphatase CD45" JOURNAL OF MEDICINAL CHEMISTRY, vol. 44, 2001, pages 1777-93, XP001014517

## Description

Compounds, compositions and their use as medicaments for the treatment of immunologically-related diseases and disorders such as autoimmune disorders and organ graft rejection.

### Related Art

Action of the immune system is known to be involved in immunologically-related diseases and disorders such as autoimmune disorders and in organ graft rejection ("OGR"). Hematopoietic, thymus-derived cells, (so-called "T cells") have an important and pervasive role as regulators and effectors of the functions of the immune system. Hematopoietic cells, and T cells in particular have on their surfaces a major transmembrane glycoprotein designated CD45, characterized by a cluster of antigenic determinants. CD45 is also known as leukocyte common antigen ("LCA"). The cytosolic portion of CD45 has protein tyrosine phosphatase ("PTP") activity and CD45 activity is known to be essential for TCR initiated T cell activation. Studies in CD45-deficient cell lines have shown that CD45 is a positive regulator of the T-Cell Receptor ("TCR") and that CD45 functions in TCR regulation by dephosphorylating the src kinases p56^{*lck*} and p59^{*fyn*}, which allows autophosphorylation of the positive regulatory site on these enzymes; these reactions lead to downstream events and ultimately to T cell activation. United States patent 5,866,569 describes nitric oxide synthetase inhibitors useful for the treatment of inflammation and autoimmune diseases which are naphthalene-1,2-diones substituted at the 4-position by an amino group bearing alkyl, aryl or arylalkyl substituents.

Available treatments for autoimmune disorders and OGR have therapeutic disadvantages. For example, Cyclosporin A, the drug most commonly used to treat OGR, has renal and CNS toxicity.

### Summary of the Invention

Potent inhibitors of CD45 have been discovered. Such inhibitors are useful for the treatment of various autoimmune disorders as well as for treatment of OGR. Inhibition of the phosphatase activity of CD45 by compounds of the present invention has been shown by incubating the cytosolic portion of CD45 with the compounds and *p*-nitrophenyl phosphate (*p*NPP), a phosphatase substrate. Spectrophotometric monitoring has shown that the liberation of *p*-nitrophenol from the substrate by CD45 is inhibited in the presence of the compounds disclosed herein. Inhibition of the phosphatase activity of CD45 by compounds of the present invention has also been shown using a p56^{*lck*} carboxy-terminal phosphorylated peptide as a substrate. Compounds of the present invention have also been shown to inhibit proliferation of T cells in a T-cell proliferation assay.

Compounds of the present invention are naphthalenediones in accord with structural diagram I: 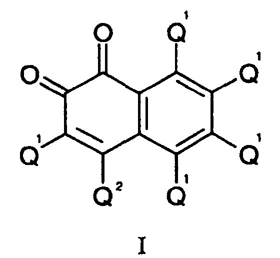 wherein:
Q¹ at each occurrence is independently selected from hydrogen, hydroxy, halogen, C(O)O(C₁-C₃)alkyl and C(O)phenyl, and
Q² is selected from hydrogen, halogen, O-(C₁-C₃)alkyl, O-(C₁-C₃)alkenyl, phenyl, indolyl and naphthyl, where phenyl may be mono- or di-substituted with NO₂ or halogen, and indolyl may be substituted with (C₁-C₃)alkyl or phenyl.

Particular compound of the invention are 4-(4-bromo-phenyl)-[1,2]naphthoquinone; 4-(3,5-dichloro-phenyl)-[1,2]naphthoquinone; 4-(3-nitro-phenyl)-[1,2]naphthoquinone; 5,6-dioxo-5,6-dihydro-naphthalene-1-carboxylic acid methyl ester, and 5,6-dioxo-5,6-dihydronaphthalene-2-carboxylic acid methyl ester.

Compounds of the present invention are ligands of CD45 which, when bound, inhibit the activity of the protein tyrosine phosphatase (PTP) activity of the cytosolic portion of CD45. Binding of a compound of the present invention to CD45 inhibits the activity of CD45 essential for TCR initiated T cell activation. Thus, compounds of the invention inhibit the positive regulation of the TCR that leads to downstream events and T cell activation. Compounds of the present invention are useful to suppress the action of the immune system in immunologically-related diseases and disorders such as autoimmune disorders and organ graft rejection and to inhibit the action of T cells as functional regulators and effectors of the immune system.

The present invention also encompasses compositions made with compounds described herein useful for the treatment of immunologically-related diseases and disorders and methods utilizing such compositions for treating such disorders.

### Detailed Description of the Invention

As used herein, (C₁-C₄)alkyl has its conventionally-understood meaning and particularly means linear or branched hydrocarbon chains having from one to four carbon atoms and thus includes methyl, ethyl, propyl, isopropyl, n-butyl, sec-butyl, isobutyl, tert-butyl, and the like.

As used herein, halo(C₁-C₄)alkyl has its conventionally-understood meaning and particularly means (C₁-C₄)alkyl as used herein wherein hydrogen atoms have been replaced by halogen atoms and thus includes monochloromethyl, trifluoromethyl, difluoroethyl, trifluoropropyl, perfluoro(C₁-C₄)alkyl, and the like.

As used herein, perfluoro(C₁-C₄)alkyl has its conventionally-understood meaning and particularly means (C₁-C₄)alkyl as used herein wherein each hydrogen atom has been replaced by a fluorine atom and thus includes trifluoromethyl.

As used herein, (CH₂)ₙ has its conventionally-understood meaning and particularly means linear hydrocarbon chains having from one to n carbon atoms and thus includes methylene, ethylene, propylene, n-butylene groups, and the like.

As used herein, the terms halogen, halo, or halide have their conventionally-understood meanings and particularly mean chlorine, bromine, iodine or fluorine.

As used herein, the term "from the range 1 to 6" or the like, means any integral value in the stated range, in this example 1, 2, 3, 4, 5 or 6.

### Definitions of terms:

DMF, *N*,*N*-dimethylformamide; THF, tetrahydrofuran; TLC, thin-layer chromatography; NMR, nuclear magnetic resonance; TFA, trifluoroacetic acid; HPLC, high performance liquid chromatography; DMAP, 4-dimethylaminopyridine; DMSO, dimethylsulfoxide; IC₅₀, concentration giving 50% inhibition; CC₅₀, concentration giving 50% cytotoxicity; ND, not determined.

HPLC method used: Analytical HPLC using an HP 1100 HPLC, with a C₁₈ Dynamax column (5 cm x 4.6 mm, 3 µM particle size, 100 Å pore size), flow rate of 0.5 mL/min, 20%-60% CH₃CN in H₂O over 7.5 min, holding at 60% CH₃CN for 2.5 min, while monitoring at 254 and 210 nm.

### Examples

Example 1: [1,2]-Naphthoquinone was purchased from Acros Organics and used as received.

### Examples 2 to 4:

The compounds of examples 2 to 4 were prepared substantially in accordance with the procedures disclosed in Takuwa, A.; Soga, O.; Iwamoto, H.; Maruyama, K. *Bull. Chem. Soc. Jpn*. **1986,** *59*, 2959-2961, which procedures are incorporated herein by reference. The physical properties of the compounds are disclosed in the reference.

### Example 2: 4-Ethoxy-[1,2]naphthoquinone.

### Example 3: 4-Methoxy-[1,2]naphthoquinone.

### Example 4: 4-Allyloxy-[1,2]naphthoquinone.

### Examples 5 and 6:

The compounds of examples 5 and 6 were prepared substantially in accordance with the procedures disclosed in Perumal, P. T.; Bhatt, M. V. *Synthesis* **1980,** 943-945, which procedures are incorporated herein by reference. The physical properties of the compounds are disclosed in the reference.

### Example 5: 4-Chloro-[1,2]naphthoquinone.

### Example 6: 4-Bromo-[1,2]naphthoquinone.

### Examples 7 and 8:

The compounds of examples 7 and 8 were prepared substantially in accordance with the procedures disclosed in Henrion, J.-C.; Jacquet, B.; Hocquaux, M.; Barre, G.; Hedayatullah, M.; Lion, C. *Bull. Soc. Chim. Belg.* **1996,** *105*, 415-418, which procedures are incorporated herein by reference. The physical properties of the compounds are disclosed in the reference.

### Example 7: 4-(1-Methyl-1H-indol-3-yl)-[1,2]naphthoquinone.

### Example 8: 4-(2-Phenyl-1H-indol-3-yl)-[1,2]naphthoquinone.

### Example 9: 4-(2-Chloro-phenyl)-[1,2]naphthoquinone:

To a solution of 4-bromo-[1,2]naphthoquinone (350 mg, 1.48 mmol) in THF (20 mL) and H₂O (5 mL) was added 2-chlorophenylboronic acid (231 mg, 1.48 mmol), followed by tri-*o*-tolylphosphine (45 mg, 148 µmol) and K₂CO₃ (614 mg, 4.44 mmol). The mixture was deoxygenated with bubbling N₂ for about ten minutes, at which time the N₂ line was removed and *tris*(dibenzylideneacetone)dipalladium(0) (68 mg, 74 µmol) was added. The resultant mixture was heated to reflux for 2 hours under N₂, at which point no starting bromide was detectable by TLC (hexanes:ethyl acetate, 1:1, v/v). The mixture was cooled to room temperature and the THF evaporated under reduced pressure. The dried material was dissolved in ethyl acetate, washed sequentially with saturated aqueous ammonium chloride, H₂O and brine, dried over Na₂SO₄, filtered, and evaporated under reduced pressure. The isolated material was chromatographed on silica gel (hexanes-ethyl acetate, 4:1, v/v) and dried to yield the product, 4-(2-chloro-phenyl)-[1,2]naphthoquinone, as an orange solid. ¹H NMR (300 MHz, CDCl₃) δ 8.21 (1H, dd, *J* = 6.9, 2.1 Hz), 7.56-7.52 (3H, m), 7.48-7.44 (2H, m), 7.44 (1H, m), 6.91 (1H, dd, *J* = 2, 6 Hz), 6.39 (1H, s); HPLC: 7.45 min.

Compounds of examples 10 to 14 inclusive were made by the method of Example 9, by utilizing the appropriate boronic acid.

### Example 10: 4-(4-Bromo-phenyl)-[1,2]naphthoquinane:

Orange solid; ¹H NMR (300 MHz, CDCl₃) δ 8.22 (1H, dd, *J* = 1.8, 7.2 Hz), 7.67 (2H, d, *J =* 6.6 Hz), 7.63-7.60 (2H, m), 7.33 (2H, d, *J =* 6.6 Hz), 7.25 (1H, dd, *J =* 2, 8 Hz), 6.41 (1H, s); HPLC: 8.25 min.

### Example 11: 4-Phenyl-[1,2]naphthoquinone:

Orange-red solid; ¹H NMR (300 MHz, CDCl₃) δ 8.22 (1H, dd, *J* = 1.8, 7.2 Hz), 7.62-7.52 (5H, m), 7.46-7.44 (2H, m), 7.30 (1H, dd, *J* = 1.2, 7.5 Hz), 6.43 (1H, s); HPLC: 6.90 min

### Example 12: [1,1']Binaphthalenyl-3,4-dione:

Orange solid; ¹H NMR (300 MHz, CDCl₃) δ 8.40 (1H, dd, *J* = 1.5, 7.5 Hz), 8.00 (1H, d, *J* = 8.1 Hz), 7.96 (1H, d, *J* = 8.1 Hz), 7.72 (1H, d, *J =* 8.4 Hz), 7.62 (1H, d, *J* = 7.2 Hz), 7.57 (1H, d, *J* = 7.8 Hz), 7.53-7.42 (4H, m), 6.83 (1H, d, *J* = 8.3 Hz), 6.55 (1H, s); HPLC: 8.27 min.

### Example 13: 4-(3,5-Dichloro-phenyl)-[ 1,2]naphthoquinone:

Orange solid; ¹H NMR (300 MHz, CDCl₃) δ 8.23 (1H, dd, *J=* 1.8, 7.2 Hz), 7.67-7.56 (2H, m), 7.53 (1H, m), 7.41 (1H, m), 7.33 (1H, m), 7.20 (1H, d, *J* = 7.5 Hz), 6.40 (1H, s); HPLC: 9.0 min.

### Example 14: 4-(3-Nitro-phenyl)-[1,2]naphthoquinone:

Orange solid; ¹H NMR (300 MHz, CDCl₃) δ 8.41 (1H, d, *J* = 7.2 Hz), 8.34 (1H, s), 8.27 (1H, m), 7.78 (2H, m), 7.63 (2H, m), 7.15 (1H, d, *J* = 7.7 Hz), 6.46 (1H, s); HPLC: 5.78 min.

### Examples 15 to 17:

The compounds of examples 15 to 17 were prepared substantially in accordance with the procedures disclosed in Barton, D.H.R.; Brewster, A.G.; Ley, S.V.; Read, C.M.; Rosenfeld, M.N. *J. Chem. Soc. Perkin Trans. I* **1981**, 1473-1476, which procedures are incorporated herein by reference.

### Example 15: 6-Benzoyl-[1,2]naphthoquinone:

Orange solid; ¹H NMR (300 MHz, CDCl₃) δ 8.23 (1H, d, *J* = 7.8 Hz), 7.86 (1H, dd, *J* = 1.5, 7.8 Hz), 7.83-7.79 (3H, m), 7.67 (1H, m), 7.57-7.51 (3H, m), 6.54 (1H, d, *J =* 10.2 Hz); HPLC: 5.93 min.

### Example 16: 5,6-Dioxo-5,6-dihydro-naphthalene-1-carboxylic acid methyl ester:

This compound was prepared using 5-carbomethoxy-2-naphthol as a starting material. The starting material was prepared according to the method of Anderson, L.C.; Thomas, D.G. *J. Am. Chem. Soc.* **1943,** *65*, 234-238. Orange solid; ¹H NMR (300 MHz, CDCl₃) δ 8.62 (1H, d, *J* = 11 Hz), 8.29 (1H, d, *J* = 7.8 Hz), 8.20 (1H, d, *J* = 7.8 Hz), 7.58 (1H, t, *J =* 7.8 Hz), 6.56 (1H, d, *J* = 11 Hz), 3.99 (3H, s); HPLC: 3.69 min.

### Example 17: 5,6-Dioxo-5,6-dihydro-naphthalene-2-carboxylic acid methyl ester:

This compound was prepared using 6-carbomethoxy-2-naphthol as a starting material. The starting material was prepared according to the method of Anderson, L.C.; Thomas, D.G. *J. Am. Chem. Soc.* **1943**, *65*, 234-238. Orange solid; ¹H NMR (300 MHz, CDCl₃) δ 8.17 (2H, m), 8.05 (1H, s), 7.52 (1H, d, *J* = 10.2 Hz), 6.53 (1H, d, *J* = 10.2 Hz), 3.99 (3H, s); HPLC: 3.61 min.

### Example 18: 7-Hydroxy-[1,2]naphthoquinone:

This compound was prepared substantially in accordance with the procedures disclosed in Teuber, H.-J.; Gotz, N. *Chem. Ber.* **1954,** 1236-1251, which procedures are incorporated herein by reference. Red solid; ¹H NMR (300 MHz, DMSO-d₆) δ 10.50 (1H, broad s), 7.56 (1H, d, *J* = 9.8 Hz), 7.40 (1H, d, *J* = 8.1 Hz, 7.31 (1H, d, *J* = 2.7 Hz), 7.05 (1H, dd, *J =* 8.1, 2.7 Hz), 6.16 (1H, d, *J =* 9.9 Hz); Anal. Calcd. For C₁₀H₆O₃-0.1H₂O: C, 68.26; H, 3.55. Found: C 67.96, 67.89; H, 3.64, 3.65.

### ASSAYS FOR BIOLOGICAL ACTIVITY

### Method A:

### Phosphatase assay using pNPP as substrate:

CD45 enzyme was obtained from BIOMOL (Plymouth Meeting, PA). Phosphatase activity was assayed in a buffer containing final concentrations of 25 mM imidazole at pH 7.0, 50 mM NaCl, 2.5 mM ethylenediaminetetraacetic acid ("EDTA"), 5 mM dithiothreitol ("DTT") and 10 µg/mL bovine serum albumin ("BSA") using *p*NPP as a substrate. Compounds were tested in a range from 30 to 0.01 µM, with a final concentration of 1 or 5% dimethylsulfoxide ("DMSO"), depending on the compound solubility. Activity was measured by following the increase in absorbance at 405 nm using a SpectraMax Plus spectrophotometric plate reader (Molecular Devices, Sunnyvale, CA).

### Method B:

### Cytotoxicity Assay:

Calcein-AM (Molecular Probes, Eugene, OR) uptake, as a quantitative measure of cell viability, was used to evaluate the toxic effect of compounds on T cells. Briefly, PBMC were treated for 3-7 days with 3-10 µg/ml PHA, a potent T-cell mitogen, to preferentially expand the T-cell population. (Bradley, Linda M. *Cell Poliferation* in *Selected Methods in Cellular Immunology,* Eds. Mishell, B.B. and Shiigi, S.M., W.H. Freeman and Co., San Francisco, 1980.)

The T-cell lymphoblasts were purified by separation over Lymphoprep, plated at 2 x 10⁵/well in a round bottom 96-well plate containing RPMI with compound and incubated overnight at 37 °C in an incubator containing 5% CO₂. The dilution scheme and culture media were the same as those used in the T-cell proliferation assay. After the incubation period, cells were washed with Dulbecco's phosphate- buffered saline (D-PBS) and incubated with 1µM Calcein-AM for 30-45 min in D-PBS as described in the technical sheet provided with The LIVE/DEAD Viability/Cytotoxicity Kit from Molecular Probes. Percent viability was assessed on a fluorescent plate reader (excitation filter 485/20 nm; emission filter 530/25 nm) where the 100% control value is the fluorescence intensity observed in the absence of test compound.

### Method C:

### Phosphatase assay using lck 10-mer as substrate:

Phosphatase activity was assayed in 96 well plates in a buffer containing final concentrations of 25 mM HEPES at pH 7.2, 5 mM DTT and 10 µg/mL BSA, using the *lck* carboxy-terminal peptide TEGQpYQPQP as the substrate (Cho, H., Krishnaraj, R., Itoh, M., Kitas, E., Bannwarth, W., Saito, H., Walsh, C.T. 1993. Substrate specificities of catalytic fragments of protein tyrosine phosphatases (HPTPb, LAR, and CD45) toward the phosphotyrosylpeptide substrates and thiophosphotyrosylated peptides as inhibitors. *Protein* *Science* 2:977-984). Compounds were tested in a range from 30 to 0.01 µM in a final concentration of 5% DMSO. Enzyme was incubated with substrate, with or without compound, at room temperature for 1.5 h. At the end of the incubation period, BIOMOL "Green Reagent" (BIOMOL, Plymouth Meeting, PA) was added to each well, the plates incubated at room temperature for 30 min and absorbance read at 620 nm.

### Method D:

### Cell isolation and T cell proliferation assay:

Whole blood was obtained from healthy human blood donors. Peripheral blood mononuclear cells ("PBMC") were isolated using Lymphoprep density-gradient centrifugation (Nycomed Amersham, Oslo, Norway), washed, counted and resuspended at 2 X 10⁶ cells/mL in RPMI 1640 medium containing glutamine, 0.1 mg/mL gentamycin and 10% heat inactivated human serum. PBMC were transferred to 96-well plates (2 X 10⁵ cells/well) containing compound or vehicle control, with the final concentration of DMSO not to exceed 0.3%, and incubated for 1 hour before addition of the activating anti-CD3 antibody, OKT3 (30 ng/mL). After 24 hours in culture, the cells were pulsed with [³H]thymidine (1 µCi/well) overnight and harvested the next day onto 96-well Packard GF/C filter plates using a Packard Cell Harvester (Packard Instruments, Meriden, CT). The filter plate was dried, the bottom of the plate sealed, 25 µL of Microscint 20 scintillation fluid added to each well, the top of the plate sealed with TopSeal-A, and the plate counted on a Packard TopCount. The data from the TopCount is transferred into Excel 5 (Microsoft, Redmond, WA) and formatted for EC₅₀ determination using Prism software (GraphPad Software, San Diego, CA).

Table 1 shows the inhibition of CD45 activity in the *p*NPP asssay and the *lck* assay certain compounds of the present invention. Inhibition in the T cell proliferation assay, as well as results from T cell cytotoxicity assay are shown.

**Table 1:**

| **Example. No.** | **pNPP IC**_{**50**} **(µM)** | **lck IC**_{**50**} **(µM)** | **T cell prolif. IC**_{**50**} **(µM)** | **CC**_{**50**} **(µM)** |
|---|---|---|---|---|
| 1 | 3 | >30 | 7 | >30 |
| 2 | 2 | ND | >30 | 30 |
| 3 | 1.2 | 4.2 | >30 | ND |
| **4** | 1.2 | 4.9 | >30 | >30 |
| **5** | 10 | >30 | >30 | >30 |
| 6 | 8.7 | >30 | 22 | >30 |
| **7** | 5 | 19 | 1.5 | >30 |
| **8** | 2.9 | 20 | 2 | 16 |
| **9** | 8 | >30 | 0.3 | 12.4 |
| **10** | 7.7 | >30 | 0.11 | 3 |
| **11** | 5 | >30 | 0.15 | 12 |
| **12** | 22.5 | >30 | 3.2 | >30 |
| **13** | 5.5 | ND | 0.3 | 5.5 |
| **14** | 7.9 | >30 | 0.2 | 4 |
| **15** | 22 | >30 | 11 | >30 |
| **16** | 11 | >30 | 3.5 | >30 |
| **17** | 7 | >30 | 7 | >30 |
| **18** | 4.9 | ND | >30 | >30 |

## Claims

1. The use of a compound in accord with structural diagram I: 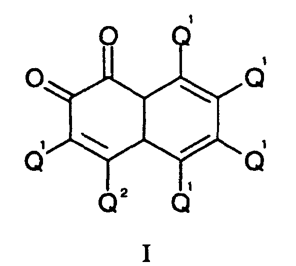 or tautomers thereof or pharmaceutically-acceptable salts thereof for the manufacture of a medicament for treating immunologically-related diseases, autoimmune disorders and organ graft rejection:
wherein:
Q¹ at each occurrence is independently selected from hydrogen, hydroxy, halogen, C(O)O(C₁-C₃)alkyl and C(O)phenyl.
Q² is selected from hydrogen, halogen, O-(C₁-C₃)alkyl, O-(C₁-C₃)alkenyl, phenyl, indolyl and naphthyl, where phenyl may be mono- or di-substituted with NO₂ or halogen, and indolyl may be substituted with (C₁-C₃)alkyl or phenyl.

2. The use of a compound according to Claim 1, wherein in a compound in accord with structural diagram I:
Q² is selected from O-methyl, O-ethyl, O-CH₂CH=CH₂, 3-(N-methyl-indole), 3-(2-phenyl-indole), 1-naphthyl, phenyl, 2-chlorophenyl, 4-bromophenyl, 3,5-dichlorophenyl and 3-nitrophenyl, and
Q¹ is hydrogen.

3. A pharmaceutical composition for use in the manufacture of a medicament of Claim 1, comprising a pharmaceutically-acceptable excipient or diluent together with a compound in accord with structural diagram I: 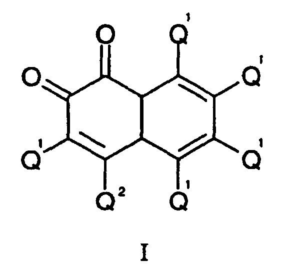 or tautomers thereof or pharmaceutically-acceptable salts thereof: wherein:
Q¹ at each occurrence is independently selected from hydrogen, hydroxy, halogen, C(O)O(C₁-C₃)alkyl and C(O)phenyl.
Q² is selected from hydrogen, halogen, O-(C₁-C₃)alkyl, O-(C₁-C₃)alkenyl, phenyl, indolyl and naphthyl, where phenyl may be mono- or di-substituted with NO₂ or halogen, and indolyl may be substituted with (C₁-C₃)alkyl or phenyl.

4. A compound selected from:
4-(4-bromo-phenyl)-[1,2]naphthoquinone;
4-(3,5-dichloro-phenyl)-[1,2]naphthoquinone;
4-(3-nitro-phenyl)-[ 1,2]naphthoquinone;
5,6-dioxo-5,6-dihydro-naphthalene-1-carhoxylic acid methyl ester, and
5,6-dioxo-5,6-dihydro-naphthalene-2-carboxylic acid methyl ester.

5. A pharmaceutical composition comprising an effective amount of a compound according to Claim 4, and a pharmaceutically-acceptable excipient or diluent.

6. The use of a compound according to Claim 4 for the manufacture of a medicament for use in-treating immunologically-related diseases, autoimmune disorders and organ graft rejection.

## Patentansprüche

1. Verwendung einer Verbindung gemäß dem Strukturdiagramm I: 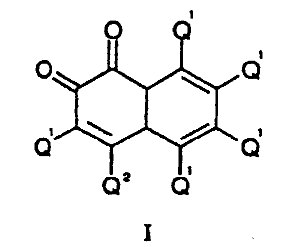 oder von Tautomeren davon oder pharmazeutisch annehmbaren Salzen davon zur Herstellung eines Medikaments zur Behandlung immunologisch assoziierter Erkrankungen, Autoimmunkrankheiten sowie der Abstoßung von Organtransplantaten:
wobei:
Q¹ jeweils unabhängig ausgewählt ist aus Wasserstoff, Hydroxy, Halogen, C(O)O-Alkyl(C₁-C₃) und C(O)Phenyl,
Q² ausgewählt ist aus Wasserstoff, Halogen, O-Alkyl(C₁-C₃), O-Alkenyl(C₁-C₃), Phenyl, Indolyl und
Naphtyl, wobei Phenyl mit NO₂ oder Halogen einfach oder zweifach substituiert und Indolyl mit (C₁-C₃)Alkyl oder Phenyl substituiert sein kann.

2. Verwendung einer Verbindung nach Anspruch 1, wobei in einer Verbindung gemäß dem Strukturdiagramm I:
Q² ausgewählt ist aus O-Methyl, O-Ethyl, O-CH₂CH=CH₂, 3-(N-Methylindol), 3-(2-Phenylindol), 1-Naphtyl, Phenyl, 2-Chlorphenyl, 4-Bromphenyl, 3,5-Dichlorphenyl und 3-Nitrophenyl, und
Q¹ für Wasserstoff steht.

3. Pharmazeutische Zusammensetzung zur Verwendung bei der Herstellung eines Medikaments nach Anspruch 1, wobei die pharmazeutische Zusammensetzung einen pharmazeutisch annehmbaren Hilfsstoff oder ein pharmazeutisch annehmbares Verdünnungsmittel zusammen mit einer Verbindung gemäß dem Strukturdiagramm I: 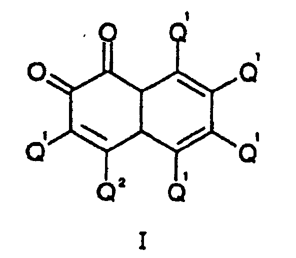 oder Tautomeren davon oder pharmazeutisch annehmbaren Salzen davon:
wobei:
Q¹ jeweils unabhängig ausgewählt ist aus Wasserstoff, Hydroxy, Halogen, C(O)O-Alkyl(C₁-C₃) und C(O)Phenyl,
Q² ausgewählt ist aus Wasserstoff, Halogen, O-Alkyl(C₁-C₃), O-Alkenyl(C₁-C₃), Phenyl, Indolyl und
Naphtyl, wobei Phenyl mit NO₂ oder Halogen einfach oder zweifach substituiert und Indolyl mit (C₁-C₃)Alkyl oder Phenyl substituiert sein kann, umfaßt.

4. Verbindung, ausgewählt aus:
4-(4-Bromphenyl)[1,2]naphtochinon;
4-(3,5-Dichlorphenyl)[1,2]naphtochinon;
4-(3-Nitrophenyl)[1,2]naphtochinon;
5,6-Dioxo-5,6-dihydronaphtalin-1-carbonsäuremethylester, und
5,6-Dioxo-5,6-dihydronaphtalin-2-carbonsäuremethylester.

5. Pharmazeutische Zusammensetzung, umfassend eine wirksame Menge einer Verbindung nach Anspruch 4 sowie einen pharmazeutisch annehmbaren Hilfsstoff oder ein pharmazeutisch annehmbares Verdünnungsmittel.

6. Verwendung einer Verbindung nach Anspruch 4 zur Herstellung eines Medikaments zur Verwendung bei der Behandlung immunologisch assoziierter Erkrankungen, Autoimmunkrankheiten sowie der Abstoßung von Organtransplantaten.

## Revendications

1. Utilisation d'un composé conforme au diagramme structural I : 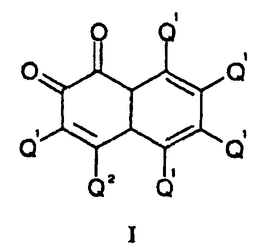 ou de tautomères de celui-ci ou de sels pharmaceutiquement acceptables de celui-ci pour la fabrication d'un médicament destiné au traitement de maladies immunologiques, de troubles auto-immunes et de rejet de greffe d'organe ;
dans laquelle
Q¹ à chaque occurrence est choisi indépendamment parmi hydrogène, hydroxy, halogène, C(O)O(alkyl(C₁-C₃) et C(O)phényle ;
Q² est choisi parmi hydrogène, halogène, O-(alkyl(C₁-C₃), O-alcényl(C₁-C₃), phényle, indolyle et naphtyle, où phényle peut être mono- ou disubstitué par NO₂ ou halogène, et indolyle peut être substitué par alkyl(C₁-C₃) ou phényle.

2. Utilisation d'un composé selon la revendication 1, dans laquelle dans un composé conforme au diagramme structural I :
Q² est choisi parmi O-méthyle, O-éthyle, O-CH₂CH=CH₂, 3-(N-méthylindole), 3-(2-phénylindole), 1-naphtyle, phényle, 2-chlorophényle, 4-bromophényle, 3,5-dichlorophényle et 3-nitrophényle, et
Q¹ est hydrogène.

3. Composition pharmaceutique pour une utilisation dans la fabrication d'un médicament selon la revendication 1, comprenant un excipient ou diluant pharmaceutiquement acceptable conjointement avec un composé conforme au diagramme structural I : 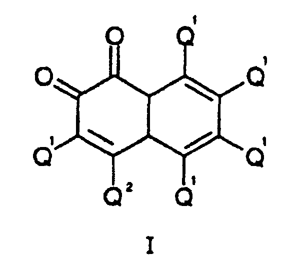 ou des tautomères de celui-ci ou des sels pharmaceutiquement acceptables de celui-ci :
dans laquelle
Q¹ à chaque occurrence est choisi indépendamment parmi hydrogène, hydroxy, halogène, C(O)O(alkyl(C₁-C₃) et C(O)phényle ;
Q² est choisi parmi hydrogène, halogène, O-(alkyl(C₁-C₃), O-alcényl(C₁-C₃), phényle, indolyle et naphtyle, où phényle peut être mono- ou disubstitué par NO₂ ou halogène, et indolyle peut être substitué par alkyl(C₁-C₃) ou phényle.

4. Composé choisi parmi :
la 4-(4-bromophényl)[1,2]naphtoquinone ;
la 4-(3,5-dichlorophényl)[1,2]naphtoquinone ;
la 4-(3-nitrophény)[1,2]naphtoquinone ;
l'ester méthylique de l'acide 5,6-dioxo-5,6-dihydronaphtalène-1-carboxylique ; et
l'ester méthylique de l'acide 5,6-dioxo-5,6-dihydronaphtalène-2-carboxylique.

5. Composition pharmaceutique comprenant une quantité efficace d'un composé selon la revendication 4, et un excipient ou diluant pharmaceutiquement acceptable.

6. Utilisation d'un composé selon la revendication 4, pour la fabrication d'un médicament pour une utilisation destinée au traitement de maladies immunologiques, de troubles auto-immunes et de rejet de greffe d'organe.
